# EUROPEAN PATENT SPECIFICATION

(11) **EP 2 008 655 B1**
(45) Date of publication and mention of the grant of the patent: **26.06.2013**
(21) Application number: 07734226.9
(22) Date of filing: 10.04.2007
(51) Int. Cl.: A61K 31/405, A61K 31/51, A61K 31/4415, A61K 31/714, A61P 29/00, A61K 31/407

(54) **Pharmaceutical composition that comprises an analgesic and vitamins**
Pharmazeutische zusammensetzung mit einem analgetikum und vitaminen
Composition pharmaceutique comprenant un analgesique et des vitamines

(30) Priority: 10.04.2006 MX PA06004020
(43) Date of publication of application: 31.12.2008
(73) Proprietor: Laboratorios Senosiain, S.a. De C.V., Distrito Federal 11560 (MX)
(72) Inventor: GARCÍA-SALGADO LOPEZ, Enrique Raúl, Distrito Federal 54020 (MX); BARRANCO HERNÁNDEZ, Gustavo, Distrito Federal 06720 (MX)
(74) Representative: Carpintero Lopez, Francisco
(86) International application number: PCT/IB2007/000907
(87) International publication number: WO 2007/116287

(56) References cited:
- WO-A1-99/53928
- FR-M- 4 492
- US-A- 4 564 614
- US-A1- 2003 191 187
- US-A1- 2005 232 869
- BROWN C R ET AL: "Analgesic efficacy and safety of single-dose oral and intramuscular ketorolac tromethamine for postoperative pain." PHARMACOTHERAPY 1990, vol. 10, no. 6 ( Pt 2), 1990, pages 59S-70S, XP009120353 ISSN: 0277-0008
- ROOKS W H 2ND ET AL: "The analgesic and anti-inflammatory profile of ketorolac and its tromethamine salt." DRUGS UNDER EXPERIMENTAL AND CLINICAL RESEARCH 1985, vol. 11, no. 8, 1985, pages 479-492, XP009120368 ISSN: 0378-6501
- REYES GARCÍA G ET AL: "Analgesic effect of sodium diclofenac plus vitamin B complex in the PIFIR model." PROCEEDINGS OF THE WESTERN PHARMACOLOGY SOCIETY 1999, vol. 42, 1999, pages 91-92, XP002538016 ISSN: 0083-8969
- MEDINA-SANTILLAN R. ET AL.: 'B Vitamins increase the analgesic effect of Ketorolac in the formalin test in the rat' PROCEEDINGS OF THE WESTERN PHARMACOLOGY SOCIETY vol. 47, 2004, pages 95 - 99, XP009120320
- REYES G. ET AL.: 'Effect of Dexamethasone Plus Vitamin B Complex in the PIFIR Model' PROCEEDINGS OF THE WESTERN PHARMACOLOGIC SOCIETY vol. 43, 2000, pages 51 - 53, XP008130657
- MEDINA-SANTILLÁN ROBERTO ET AL: "B vitamins increase the analgesic effect of ketorolac in the formalin test in the rat", WESTERN PHARMACOLOGY SOCIETY. PROCEEDINGS, WESTERN PHARMACOLOGY SOCIETY, TUCSON, AZ, US, vol. 47, 1 January 2004 (2004-01-01), pages 95-99, XP009120320, ISSN: 0083-8969

## Description

### TECHNICAL FIELD

This invention refers to the pharmaceutical combinations of ketorolac salts and B-complex; to the methods used to make said combinations; and particularly, to ketorolac and B-complex synergic combinations useful in the treatment of patients that suffer from moderate to severe pain and neuralgias in different sites.

### BACKGROUND OF THE INVENTION

A satisfactory pain management is one of the most important symptomatologies that has not been solved completely, which causes a strong impact in patients and in the whole health system.

Pain is generated by two of these mechanisms: (a) direct mechanisms as a result of cutting nervous termination throughout all the different structures affected by the surgical aggression; and (b) indirect mechanisms consisting in the delivery of algogenic substances capable of activating and/or sensitizing the receptors in charge of processing nociceptive sensation. Through them a series of nociceptive impulses is created that, after reaching the nervous central system, they trigger a cascade reaction, affecting different systems: respiratory, cardio-circulatory, digestive, endocrine, and metabolic. The inappropriate pain management increases the post-surgical morbidity, extending hospital stay and costs.

One of the most used therapies in the immediate post-surgical period to avoid pain is by the use of parenteral analgesics such as t opioids. However, the restrictions in the use of opioids is associated with the side effects, inter alia, itching, nausea, vomiting, urinary retention, constipation, sedation, and potential respiratory depression, paralytic ileo, tolerance, anxiety and abstinence syndrome.

An alternative to the use of opioids and their side effects corresponds to the use of non-steroidal anti-inflammatory drugs (NSAID) since they improve analgesia and prevent nociceptors sensibility. Generally, most used non-steroidal anti-inflammatory drugs are: acetaminophen, indomethacin, ibuprofen, meloxicam, diclofenac and ketorolac, among others.

Likewise, the combination of a non-steroidal anti-inflammatory drug with an opioidal analgesic is known. For example in Mexican patent application No. PA/a/2002/010828, which describes capsules containing ketorolac (NSAID) and tramadol (opioid) for pain management. The disadvantage of said composition is related to the adverse effects of opiods, like: tolerance, anxiety, abstinence syndrome and/or respiratory depression.

US patent application No. 11/078,902, under publication number 20050232869 (publication date March 11th, 2005) which describes a spray foam therapeutic kit. Said composition contains a non-steroidal anti-inflammatory drug, and vitamin B or a derivative thereof may be added to the composition. The disadvantage of this composition lies in its administration, which is exclusively topic and is indicated to manage skin conditions, swelling and other inflammatory disorders. Thus, said application does not specifically disclose a formulation containing ketorolac and B-complex for a variety of administration routes.

Patent US 6,051,587 (equivalent to EP 1071430 and PCT/CA99/00331 applications) refers to a pharmaceutical composition containing an anti-inflammatory analgesic (NSAID) in combination with pyridoxine equivalent to Vitamin B6. However, said composition was developed for iatrogenic age-related hypertension treatment and is different from the composition of this invention, which contains an analgesic in a dose under the regular one used in therapy, and B-complex.

Nowadays, there is a medicine being commercialized that includes diclofenac and B-complex. One of the disadvantages of this composition lies in the risk of necrosis caused by repeated parenteral administration lasting more than two days, this situation limits the product therapy time. Diclofenac is an anti-inflammatory anti-rheumatic compound that causes adverse effects, and intestinal bleeding, among others. On the other hand, the present invention uses smaller doses of active principle than those regularly use to obtain an analgesic effect comparable to opioid compounds and there is a decrease of adverse events.

Further relevant prior art includes MEDINA-SANTILLÁN ROBERTO ET AL: "B vitamins increase the analgesic effect of ketorolac in the formalin test in the rat", WESTERN PHARMACOLOGY SOCIETY. PROCEEDINGS, WESTERN PHARMACOLOGY SOCIETY, TUCSON, AZ, US, vol. 47, 1 January 2004 (2004-01-01), pages 95-99 and US 2003191187.

In spite of what was described about the technical field, there is still the need of a pharmaceutical composition with a high therapeutic efficacy and a wide security margin. It is considered that a NSAID such as ketorolac, used in doses smaller than those regularly used in combination with the vitamin B-complex, can meet these two requirements, in addition of showing synergism between the two components in preventing moderate to severe pain and neuralgias in different sites of the body, and an important decrease of adverse effects, such as digestive bleeding among others.

The pharmaceutical composition of this invention meets the needs before described and provides advantages such as explained in the specification below.

### BRIEF DESCRIPTION OF THE DRAWINGS

The figures illustrate the behavior of drugs when administrated to patients under study.
Figure 1 shows the assessment of Ketorolac anti-nociceptive activity effect.
Figure 2 shows the assessment of B-complex anti-nociceptive activity effect.
Figure 3 shows the assessment of riboflavin anti-nociceptive activity effect.
Figure 4 shows the assessment of anti-nociceptive activity effect of ketorolac and B-complex combination.
Figure 5 shows the assessment of anti-nociceptive activity effect of ketorolac and B-complex combination.
Figure 6 shows the assessment of anti-nociceptive activity effect when administering ketorolac, riboflavin and B-complex.
Figure 7 shows the assessment of anti-nociceptive activity effect when administering ketorolac and riboflavin as well as ketorolac and B-complex.
Figure 8 shows isobolograms showing the additive effect of the ketorolac and riboflavin combination, as well as the synergic interaction of the ketorolac and B-complex combination.

### DESCRIPTION OF THE INVENTION

Almost all the so-called NSAIDs used nowadays, inhibit the activity of cyclooxygenase-1 (COX-1) present in the constitutive elements of almost every cell and mediates physiological reactions; and cyclooxygenase-2 (COX-2) present in damaged tissue needs to be induced, it is expressed in a transitory form and almost exclusively in stimulated inflammatory cells, and promotes the large and fast formation of inflammation mediators.

The anti-inflammatory activity of NSAIDs is obtained via the COX-2 inhibition in the inflammation site. These drugs are also capable of inhibiting COX-1 in gastrointestinal and renal tissues, which generates undesired effects and can limit their therapeutic utility. In other words, the risk-benefit ratio of NSAID will depend on their capability of blocking the cyclooxygenases COX forms in a greater or lesser degree.

Ketorolac, like other NSAIDs, inhibits the cyclooxygenase (COX) enzyme therefore, prostaglandin production decreases, thereby reducing the inflammatory reactions and the nociceptive transmission triggering mechanisms. It also produces central anti-nociceptive effects. By inhibiting prostaglandin formation Ketorolac decreases the cyto-protection factors, thromboxanes are inhibited causing bleedings. In the present invention, which uses only half the regular dose of ketorolac, the adverse effects are importantly reduced, which significantly beneficiates the patient without losing the therapeutic and analgesic effects of ketorolac.

Ketorolac tromethamine, (±)-5(benzoyl)-2,3-dihydro-1N-pyrrolizine-1-carboxylic acid tris hydroxy methyl amino methane is a non-steroidal anti-inflammatory drug (NSAID) that belongs to the carboxylic and pyrrol pyrrolic derivatives, which is effective in the treatment of moderate and severe pain.

Ketorolac administration in healthy humans shows a fast adsorption, both oral and intramuscular; the time to reach maximum plasma concentration (tmax) has been reported to be 53 and 46 minutes, respectively. Likewise, 92% of ketorolac is excreted in urine and 6% in feces.

The usual ketorolac dose in intramuscular parenteral form is 30 mg each 4-6 hours, oral dose is 10 mg from 1 to 4 times a day and it should not exceed 40 mg in adults.

Ketorolac is highly soluble in water. Clinical studies have shown that ketorolac is a strong and effective analgesic. In comparison with opioid analgesics, ketorolac does not produce adverse effects unlike opioids, additionally, is a moderate antipyretic. Ketorolac is one of the few non-steroidal anti-inflammatory drugs approved for parenteral administration. However, when administered in combination with other substances, it usually presents stability problems. Laboratory tests have demonstrated that its stability decreases significantly when acid solutions are present.

B-complex is composed of vitamins of group B such as: vitamin B1 (Thiamine chlorhydrate or mononitrate), vitamin B2 (Riboflavin), vitamin B3 (Niacin, Vitamin B6 (Pyridoxine Chlorhydrate), vitamin B8 (Biotin), vitamin B12 (Cyanocobalmin), vitamin B15 (Pangamic Acid or DIEDI), among others. Vitamins B may be hydro- and liposoluble. These vitamins are important to stay healthy.

In the preferred embodiment of this invention the term "B-complex" refers to the next vitamins:

Thiamine (B1): part of an enzyme that breaks-down and assimilates carbohydrates; is essential for nucleic acids, DNA, and RNA (gen carriers). It Promotes appetite and normalizes the nervous system functions, therefore, it is essential to keep the functional integrity of cardiovascular, digestive and nervous system.

Thiamine (B6): promotes the metabolism of fats and proteins and intervenes in the transformation of tryptophan, an amino acid, into niacin, stimulates the fagocitic activity of white cells.

Cyanocobalmin (B12): helps in the formation of nucleic acids, contributes to the normal functioning of red cells and helps keep nervous cell.

B-complex has multiple therapeutic applications in human beings, such as: deficiencies of vitamins which integrate this complex, in pre or post-surgical condition, neuritis, polyneuritis, chronic diarrhea, polyneuropathies and Wernicke encephalopathies. It is also used in complementary treatment for AIDS or hepatic cirrhosis patients, in neurodegenerative problems such as multiple sclerosis; in addition, B-complex has a therapeutic anti-inflammatory effect.

The Ketorolac and B-complex pharmaceutical composition of this invention is new, since in the state-of-the-art there is no disclosure of a composition with both technical aspects that characterize it. Ketorolac is a non-steroidal anti-inflammatory analgesic. B-complex possesses anti-nociceptive properties. In combination, the present invention achieves a synergic effect to treat patients suffering from moderate to severe pain and neuralgias in different body sites. It's important to emphasize that when using a ketorolac dose smaller than the therapeutic ones typically used, a reduction of adverse effects is obtained.

Ketorolac is one of the few non-steroidal anti-inflammatory analgesic approved for parenteral administration; however, it presents severe stability problems when administered in combination with other substances. In the present invention, when combining ketorolac with B-complex a stable composition is obtained, which allows this novel composition to be parenteral administrated.

It's important to mention that nowadays there are injectable pure ketorolac formulations (individual pharmaceutical form) and pure B-complex (individual pharmaceutical form) which may lead us to believe that a simple mixing of these two formulations can be done. However, this is not possible, since there is a series of physical-chemical implications for each compound. For instance, due to the physicochemical characteristics of ketorolac, it has greater stability in a pH range from neutral to slightly basic. Unlike B-complex which presents greater stability in acid pH. The above mentioned makes difficult the preparation of a pharmaceutical composition in which both components coexist without affecting each other's stability. This is why a direct combination of ketorolac and B-complex is not possible.

Currently, there are no individual pharmaceutical forms for the parenteral administration of ketorolac at 15 mg/ml and B-complex, as described in this invention.
When combining ketorolac from the existing pharmaceutical forms in the market (in different concentrations e.g., 5, 10, 15, y 20 mg/ml) with B-complex, the degradation of ketorolac starts since the pH gets too much to the acid side.

When combining a ketorolac solution at 30 mg or higher concentrations with B-complex, the solution shows evident physical instability, evidenced by cloudiness which can go up to a precipitate formation. The above situation makes impossible to simply combine ketorolac and B-complex, making it necessary to research and develop a system that provides appropriate stability characteristics for parenteral administration.

Therefore, during the development of the pharmaceutical composition of this patent application, several technical problems where solved in order to achieve stable and safe composition, since using a small dose of ketorolac an evident degradation was expected, because in combination with the acid pH of the B-complex formulation, the acid pH would prevail. However, in the pharmaceutical composition of this invention, to counteract the acid pH of B-complex via appropriate buffers for this composition, making possible to obtain a stable homogeneous composition.

Achieving the ketorolac combination and B-complex stability is a critical point of the pharmaceutical composition. Such stability was achieved making ketorolac stronger through pH adjustment to a maximum level in which it maintains unaltered its physicochemical properties by using acceptable buffer solutions for parenteral use.
Based on the foregoing, achieving a stable composition that is safe and effective, between a NSAID and B-complex represents an important technical challenge.

The pharmaceutical composition of the present invention achieves stability of a ketorolac-B-complex solution, using doses of ketorolac smaller than the typical ones of 30 mg/ml; instead, 15 mg/ml doses were used. It's important to emphasize this smaller dose does not decreases the therapeutic effect of ketorolac, since in combination with B-complex a therapeutic synergy equivalent to the effect achieved with the typical ketorolac administration of 30 mg is achieved.

Another additional advantage of this pharmaceutical composition lies in that it uses a lower volume of the solution to be administered in relation with that used for existing medicines available in the market, consisting of a combination of NSAID and B-complex. So the total volume to be administered of the composition of this invention gets to be three times lower than the typical volume currently used. The practical benefit of the foregoing is to decrease pain caused to patients when the medicine is administered IM.

In the case of a slow intravenous administration, when a constant monitoring of the patient's response is necessary, the pharmaceutical composition of the invention allows it; since it has been proved that it is physicochemical stable even when exposed to light, making its slow administration possible.

The composition of this invention due to its novel solution formulation and physicochemical stability can be intravenously administered, but previously dissolved with physiological solutions such as: saline solution of 0.9% sodium chloride, 5% dextrose solution, Hartmann solution and others.

Other advantages related to this invention are that it allows: a) the use of smaller ketorolac doses than the typical ones indicated, without decreasing the analgesic and anti-inflammatory effects; (b) a 5 or more days treatment or whatever the physician prescribes; this is achieved by the synergic therapeutic action of this combination.

The foregoing is not possible using diclofenac-B-complex formulations, since after two days it can cause tissue necrosis in intramuscular administration.

An additional benefit of this pharmaceutical composition is that it doesn't generate the adverse effects caused by opioid analgesics, such as: tolerance, anxiety, abstinence effect, respiratory disorders.

This invention is related to a pharmaceutical composition consisting of therapeutically effective amounts a SALT of ketorolac, B-complex and pharmaceutically acceptable excipients.

### Formulations

### EXAMPLE 1 : PARENTERAL FORMULATIONS.

This example discloses a pharmaceutical composition consisting of therapeutically effective amounts of a salt of ketorolac, B-complex, buffer solutions, carriers and water for injection; where said composition has a pH of 3.5 to 5.5.

The pharmaceutical composition consists of two parenteral solution units:
a) a first solution unit with therapeutically effective quantities of a ketorolac salt, water and pharmaceutically acceptable vehicles, where the pH of the solution is 7.5 to 9.5.
b) a second solution unit with therapeutically effective quantities of B-complex, pharmaceutically acceptable vehicles and water for injection, where the pH of the solution is 2.5 to 4.5.

It is important to mention that the first and second units are mixed to form a composition in solution, which is physicochemical stable for administration.

Some examples of the invention are described below:
Unit 1

| **Active ingredients and excipients.** | **Quantity in mg/ml** |
|---|---|
| Ketorolac tromethamine | 2.0 to 30.0 mg |
| Ethyl alcohol 96°GL | 85 to 115 mg |
| Anhydrous sodium dibasic phosphate | 28.5 to 38.5 mg |
| Citric acid | 1.1 to 1.5 mg |
| Water for injection | q.s. 1.0 ml |
| Total | 1.00 ml |

Unit 2

| **Active ingredients and excipients.** | **Quantity in mg/ml** |
|---|---|
| Thiamine chlorhydrate | 15 to 250 mg |
| Pyridoxine chlorhydrate | 15 to 250 mg |
| Cyanocobalmin | 0.1 to 10 mg |
| Disodium edetate | 0.08 to 0.115 mg |
| Propyl paraben | 0.068 to 0.092 mg |
| Methyl paraben | 0.52 mg to 1.08 mg |
| Sodium Hydroxide solution | 0.06 ml to 0.08 ml |
| Propylene glycol | 0.085 ml to 0.15 ml |
| Water for injection | q.s. 1.0 ml |
| Total | 1.0 ml |

### UNIT 1 PREPARATION

The following information is the preferred embodiment of the invention.

The formulation of Unit 1 is as follows:

| **Active ingredients and excipients.** | **Quantity in mg/ml** |
|---|---|
| Ketorolac | 15 mg |
| Ethyl alcohol 96°GL | 100 mg |
| Anhydrous sodium dibasic phosphate | 33.55 mg |
| Citric acid | 1.33 mg |
| Water for injection | q.s. 1.0 ml |
| Total | 1.0 ml |

The steps to prepare the formulation of unit 1 are as follows:
Weight the components of the formulation.
Pre-set a container to the size of the batch, and add 49% of water for injection.

Start stirring followed by the slow addition of ketorolac salt.

Then, add the buffer excipient. It's important to mention that the buffer is selected from anhydrous sodium dibasic phosphate, borates solution or any other pharmaceutically acceptable buffer for parenteral administration which should maintain the solution stability.

Add 30% of water for injection while still constantly stirring.

Add the anhydrous citric acid, and stir until it dissolves completely.

Add the co-solvent, and stir until the mixture becomes homogenized. It's important to mention that the co-solvent is selected from ethyl alcohol or any other pharmaceutically acceptable solvent for parenteral administration.

Take the solution to 95% of the total volume by adding water for injection.

Test the pH and, if necessary, adjust it in the range of 7.5 - 9.35 with the addition of sodium hydroxide solution 1.0 N.

Perform the assay according to the specifications mentioned in the Pharmacopoeia.

Once the product is approved, filtrate the solution through a 0.22 micron porosity membrane.

Fill up units with the obtained solution up to a volume of 1.0 ml.

### UNIT 2 PREPARATION

The following information is the preferred embodiment of the invention.

The formulation of Unit 2 is as follows:
Pharmaceutical form a.

| **Active ingredients and excipients** | **Quantity in mg/ml** |
|---|---|
| Thiamine chlorhydrate | 100 mg |
| Pyridoxine chlorhydrate | 100 mg |
| Cyanocobalmin | 1 mg |
| Disodium edetate | 0.10 mg |
| Propyl paraben | 0.08 mg |
| Methyl paraben | 0.72 mg |
| Sodium Hydroxide solution | 0.07 ml |
| Propylene glycol | 0.10 ml |
| Water for injection | q.s. 1.00 ml |
| Total | 1.00 ml |

Pharmaceutical form b.

| **Active ingredients and excipients** | **Quantity in mg/ml** |
|---|---|
| Thiamine chlorhydrate | 100 mg |
| Pyridoxine chlorhydrate | 100 mg |
| Cyanocobalmin | 5 mg |
| Disodium edetate | 0.10 mg |
| Propyl paraben | 0.08 mg |
| Methyl paraben | 0.72 mg |
| Sodium Hydroxide solution | 0.07 ml |
| Propylene glycol | 0.10 ml |
| Water for injection | q.s. 1.00 ml |
| Total | 1.00 ml |

The steps to prepare Unit 2 for each of the 2 pharmaceutical presentations a and b are the following:
1. Weight the formula components
2. Add propylene glycol in a 500-mL container.
3. Stir and slowly add the preservatives by constantly stirring until total dissolution. It's important to mention that the preferred preservatives are propyl paraben and methyl paraben. However, the invention is not limited to them, and other preservatives selected by a skilled in the art may be used.
4. In a second container of 2500-mL, add 1000 ml of water and stir.
5. Add sodium hydroxide solution 1.0 M and stir to incorporate the mixture.
6. Add disodium edetate to the mixture obtained in step 5, and stir vigorously until total dissolution.
7. The mixture obtained in step 3 is incorporated to the one obtained in step 5 under continuous stirring.
8. Add the vitamins to the mixture of step 7 and stir until total dissolution.
9. Take the solution to 95% of the total volume with water for injection.
10. Test the pH and, if necessary, adjust it within a range of 2.5 - 9.5 with sodium hydroxide solution 1.0 N.
11. Take the mixture to the total volume with water for injection.
12. Once the product is approved, filtrate the solution through a 0.22 micron membrane.
13. The solution obtained is packaged in units of 1.0 ml volume by aseptic filling.
14. Units are placed into respective boxes
15. The product is sampled and analyzed as a finished product.

### COMPARATIVE EXAMPLE 2. ORAL FORMULATION

| **Ingredient** | **Milligrams/unit** |
|---|---|
| Ketorolac tromethamine | 4.25-5.75 |
| Thiamine chlorhydrate (Vitamin B1) | 42.5-57.5 |
| Pyridoxine chlorhydrate (Vitamin 86) | 42.5-57.5 |
| Cyanocobalmin (Vitamin B12) | 0.85-1.15 |
| Inert cores 24-30 | 168.8-228.5 |
| Granulated sugar | 17-23 |
| Microcrystalline cellulose PH101 | 42.5-57.5 |
| Corn starch | 12.75-17.75 |
| Aerosil | 0.85-1.15 |
| Magnesium stearate | 2.5-3.5 |
| EDTA | 0.025-0.035 |
| Citric acid | 0.25-0.35 |
| Opadry | 10.2-13.8 |
| Polyvinyl pyrrolidone | 2.55-3.45 |
| Purified water* | g.s. |

| | |
|---|---|
| *It evaporates during process | |

The following formulation is the preferred embodiment of the invention.

| **Ingredient** | **Milligrams/unit** |
|---|---|
| Ketorolac tromethamine | 5.0 |
| Thiamine chlorhydrate (Vitamin B1) | 50 |
| Pyridoxine chlorhydrate (Vitamin B6) | 50 |
| Cyanocobalmin (Vitamin B12) | 1.0 |
| Inert cores 24-30 | 198.6 |
| Granulated sugar | 20 |
| Microcrystalline cellulose PH101 | 50 |
| Corn starch | 15 |
| Aerosil | 1.0 |
| Magnesium stearate | 3.0 |
| EDTA | 0.03 |
| Citric acid | 0.30 |
| Opadry | 12 |
| Polyvinyl pyrrolidone | 3.0 |
| Purified water* | q.s. |

It should be mentioned that the following excipients may substitute those described in the above formulation.

Microcrystalline cellulose may be substituted by lactose and any other equivalent excipient properly selected by a skilled in the art.

Corn starch may be substituted by any other kind of starch such as rice, potato, etc.

Magnesium stearate may be substituted by sodium stearyl fumarate, talcum or any other equivalent excipient properly selected by a skilled in the art.

Polyvinyl pyrrolidone may be substituted by hydroxy propyl methyl cellulose and any other equivalent excipient properly selected by a skilled in the art.

Opadry may be substituted by hydroxy propyl methyl cellulose, lustreclea or any other equivalent excipient properly selected by a skilled in the art.

The steps to prepare the oral composition are as follows:
1. Weight the components of the formula.
2. Mix following the next order avicel, aerosil, starch, sugar, ketorolac, thiamine, and pyridoxine, for about 5 minutes in a plastic bag. Add magnesium stearate and mix for about 3 more minutes.
3. Pass the product obtained in step 2 through a 30 mesh.
4. Dissolve: Citric acid, EDTA, PVP, and Cyanocobalmin in water.
5. Add spheres (inert cores) to the Glatt GPCG 1.1 equipment.
6. Add the obtained powder mixture of step 3 to the powder metered dispenser (equipment Glatt GPCT).
7. Open the fluidization current and, keeping the rotor plate spinning, add the powder from step 6 with a simultaneous aspersion of the liquid prepared in step 4. Use the filter shaker in asynchronous mode.
8. Stop airflow and the equipment. Shake the filters to allow all the dust adhered to them to fall over the processed material.
9. Resume the powder adhesion process over the spheres, using the spinning disc and the liquid aspersion to incorporate the material detached from the filters.
10. Repeat steps 9 and 10 until no powder drop is observed while shaking the filters.
11. Prepare Opadry dispersion to 12% in water.
12. Apply Opadry dispersion in rotoprocess.
13. Pass spheres through a mesh No. 12 and remove fine powder not adhered to the spheres.

### EXAMPLE 3: FACTIBIILITY TEST STUDY OF THE COMPOSITION RELATED TO PHARMACEUTICALLY ACCEPTABLE PHYSIOLOGICAL SOLUTIONS.

Compatibility tests were run to ketorolac/B-complex pharmaceutical composition in combination with pharmaceutically acceptable physiological solutions.

Solutions used were 5% dextrose, Hartmannn solution and 0.9% sodium chloride solution.

A ketorolac unit and a B-complex unit were mixed in 100 ml of each of the physiological solutions, the ketorolac concentration and the B-complex vitamins concentration, and also their pH were measured at different times (0, 3, 4, 6, 8, 24 hours); the results show that the product was stable in said physiological solutions; the ketorolac and B-complex concentrations being within the 90%-110% specification. The solutions had a pH of 4.3±0.5, showing stability in each of the physiological solutions used at each time point.

### EXAMPLE 4: PRECLINICAL STUDY ONE

Methodology: We worked with five groups of 8 rats each.

The antinociceptive effect was evaluated through the heat-stimulation model in the rat paw.

Used substances: saline solution, carrageen to produce the nociceptive effect (pain), ketorolac, riboflavin and B-complex (vitamins B1, B6 and B12).

For the doses-response curve, each group received a ketorolac dose so that 0.32, 1, 1.8, 3.2 y 10 mg/kg of weight were administrated orally. The antinociceptive effect was measured for 6 hours.

Latency time curves were plot on a graph (the time rat takes before moving Hawaii its paw to avoid the pain) as a function of time.

Saline solution was used as a negative control solution (establishing the base level) and carrageen to produce a sensitizing effect (inflammation).

The antinociceptive effect of Ketorolac, B-complex in the following ratios 100:100:1 of B1, B6 and B12 (respectively), riboflavin, the combination of ketorolac and B-complex, and combinations of ketorolac and riboflavin.

The following were administered: Riboflavin (alone), Ketorolac (alone), B-Complex (alone), Ketorolac and Riboflavin combination, and Ketorolac and B-Complex combination.

### Results:

Isobolograms were made in order to evaluate the interaction between ketorolac and B-complex using the ED25 values (effective doses) obtained from the dose-response curves for the administered drugs alone or combined. The statistic difference between the point of theoretical additive effect and the obtained experimental point was evaluated by means of Student's "t" test.

After the administration of isotonic saline solution and carrageen we can clearly observe an important sensitization produced in rats receiving carrageen, since the latency time decreases considerably.

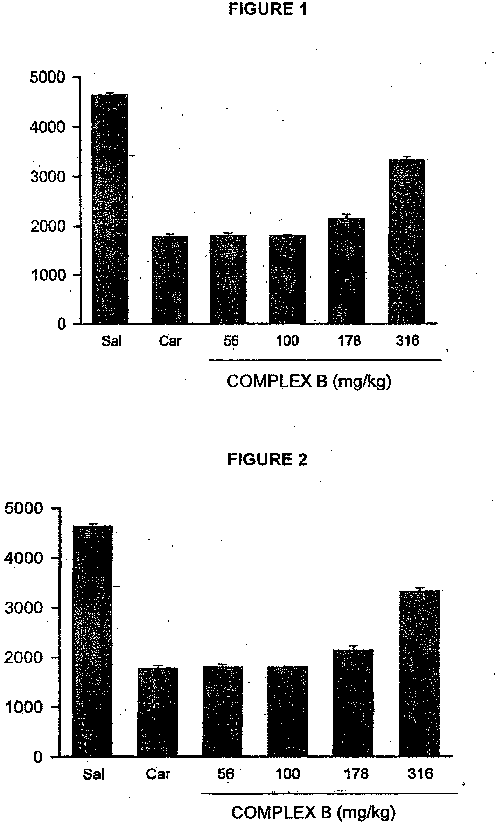

Figures 1, 2, and 3 show the dose-response curves for ketorolac, B-complex and riboflavin (respectively). In the foregoing results, we can observe that in all cases there was a dose-dependant response.

Particularly, Figure 1 shows the ketorolac dose-response curve for the heat-stimulation model in rat paw. The effect is expressed as the area under the latency curve as a function of time (Y-axis) and the X-axis indicates the ketorolac doses in mg/kg.

Particularly, Figure 2 shows the B-complex dose-response curve (vitamins B1, B6 and B12 in a 100:100:1 ratio) for the heat-stimulation model in rat paw. The effect is expressed as the area under the latency curve as a function of time (Y-axis) and the X-axis represent the B-complex dose in mg/kg.

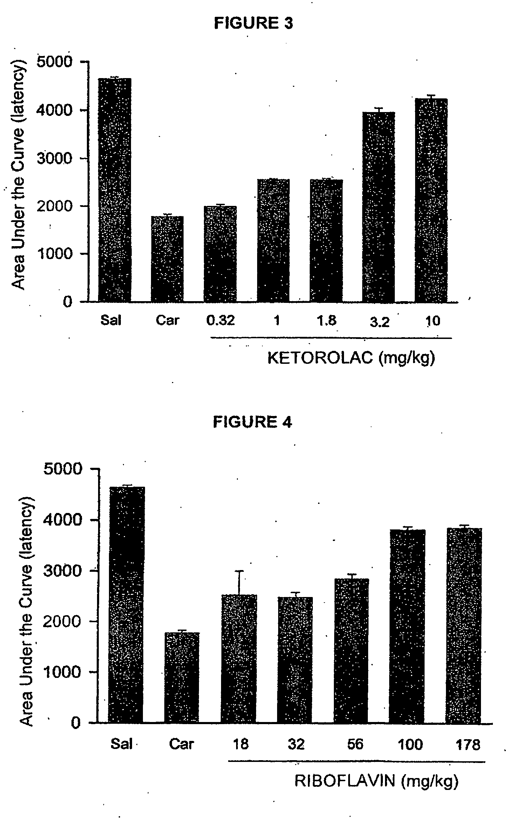

Particularly, Figure 3 shows the riboflavin dose-response curve for the heat-stimulation model in rat paw. The effect is expressed as the area under the latency curve as a function of time (Y-axis) and the X-axis represents the riboflavin dose in mg/Kg. Each bar corresponds to an average of 8 animals ± standard error.

When testing the ketorolac and B-complex combination a dose-dependant effect was observed, and it reached the level of the saline control solution (rats not receiving carrageen) indicating a surprising and important antinociceptive effect, that is, an important effect in pain decrease (See Figure 4). On the other hand, the administration of ketorolac combined with riboflavin also produced a dose-dependant effect.

Particularly, Figures 4 shows a dose-response curve fro ketorolac combined with B-complex (vitamins B1, B6 and B12 in a 100:100:1 ratio) in the heat-stimulation model in rat paw. The effect is expressed as the area under the latency curve as a function of time (Y-axis) and the Ketorolac-B-complex doses in mg/Kg.

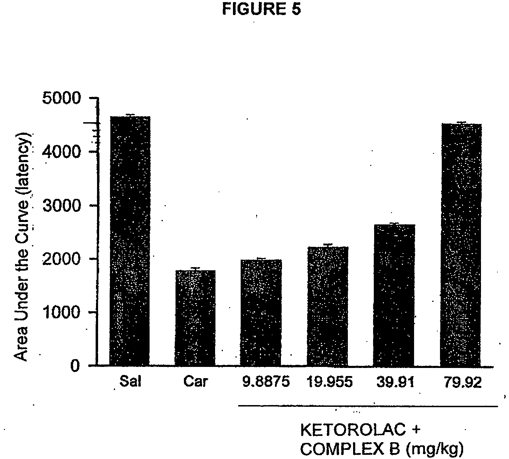

Particularly, Figure 5 shows a dose-response curve fro ketorolac combined with riboflavin for the heat-stimulation model in rat paw. The effect is expressed as the area under the latency curve as a function of time (Y-axis) and the X-axis represents Ketorolac-riboflavin doses in mg/Kg.

### Conclusions:

Based on what was mentioned above, an antinociceptive effect, the object of this study, is observed in ketorolac, B-complex, riboflavin and ketorolac-B-complex combination and ketorolac-riboflavin combination.

When associating both ketorolac with B-complex vitamins and ketorolac with riboflavin, an additive interaction in both cases was observed.

However, with respect to ketorolac and B-complex combination there was a decrease by half in the required dose. This means that in spite of not being of statistical significance it is quite clear that a lower quantity of ketorolac and vitamins is required to produce the same antinociceptive effect than that obtained with ketorolac in known doses.

From this study it can also be drawn that the administration of ketorolac and B-complex combination considerably reduces the required doses, since an antinociceptive effect is reached leading to the decrease in the probability of producing adverse effects.

### EXAMPLE 5: PRECLINICAL STUDY 2

We worked with five groups of 8 rats each. The antinociception effect was evaluated via the formalin test (1% 50 µl); rats are placed in an observation chamber made of acrylic material. The nociceptive behavior was evaluated based on the number of shakes at the injected paw.

Used substances: saline solution, formalin (produces nociceptive effect), ketorolac, riboflavin and B-complex (vitamins B1, B6 and B12).

For the dose-response curve, each group received a 0.32-10 mg/kg of ketorolac dose, 6.25-100 mg/kg of riboflavin, 56-316 mg/kg of B-complex, ketorolac-B-complex combination and ketorolac-riboflavin combinations.

The antinociceptive effect was measured for sixty minutes. The nociceptive behavior induced by formalin shows a biphasic behavior. Acute or initial phase from 0 to 10 minutes and tonic phase from 15 to 60 minutes.

Curves for the number of shakes against time were plot and the area under the curve was obtained (AUC) for the second phase, using the trapezoids method.

The dose-response curve for each composition was obtained as the percentage of the maximum possible effect.

The antinociceptive effect of ketorolac, B-complex in ratios of 100:100:1 of B1, B6 and B12 (respectively), riboflavin, ketorolac-B-complex combination, and ketorolac-riboflavin combination were evaluated.

The results were to be statistically processed by the Student's t test, and a significant value smaller than the theoretical addition value of the effective dose (p<0.05) was to be considered as a synergic interaction indicator.

A saline solution was used as a negative control solution (base level).

Administration: riboflavin (alone), ketorolac (alone), B-complex (alone), ketorolac-riboflavin combination, and ketorolac-B-complex combination.

### Results:

The administration of Formalin produced nociceptive effect.

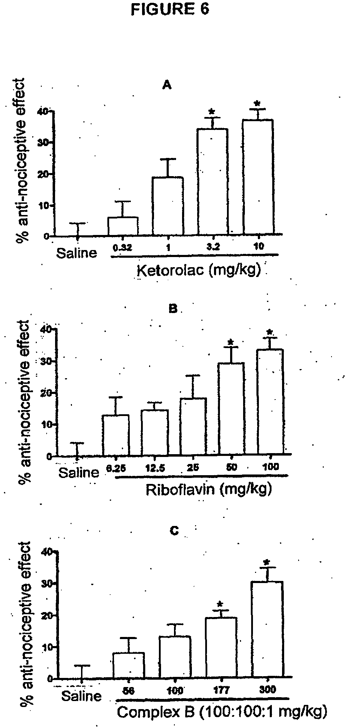

In Figure 6, the anti-nociceptive effect is observed for administered ketorolac, riboflavin and B-complex. Dose-response curves are shown, and it is observed that there is a dose-dependant response. The effect is expressed as the area under the latency curve as a function of the time; bars are the average ± standard error of mean (SEM) of at least 8 animals, where * means the significant difference to the control group (p<0.005) determined via one-way ANOVA followed by Turkey test. This test allows obtaining the difference between the means of each group, with respect to control, with its respective confidence intervals.

With the administration of B-complex a decrease in the number of dose-dependent shakes was observed for phase two, reaching a maximum effect of 29% (ED25 238.3±33.0 mg/kg). Also, riboflavin (6.25-100 mg/kg) and ketorolac (0.32-10 mg/kg) administration reduced with a dose-dependant effect, the number of shakes, reaching a maximum effect of 33% (ED25 39.8±7.1 mg/kg) for riboflavin and 36% (ED25 2.1±0.5 mg/Kg) for ketorolac.

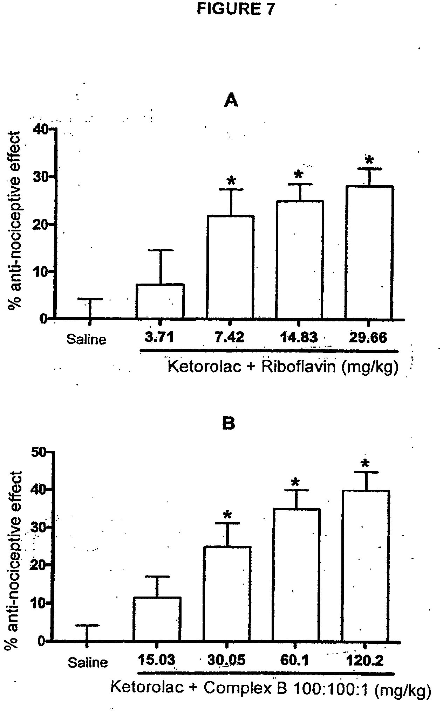

In Figure 7 the assessment of the anti-nociceptive effect is shown upon administration of ketorolac and riboflavin, as well as ketorolac and B-complex. The co-administration of ketorolac and riboflavin vitamins, as well as ketorolac and B-complex showed a dose-dependant anti-nociceptive effect in the second phase of the formalin test.

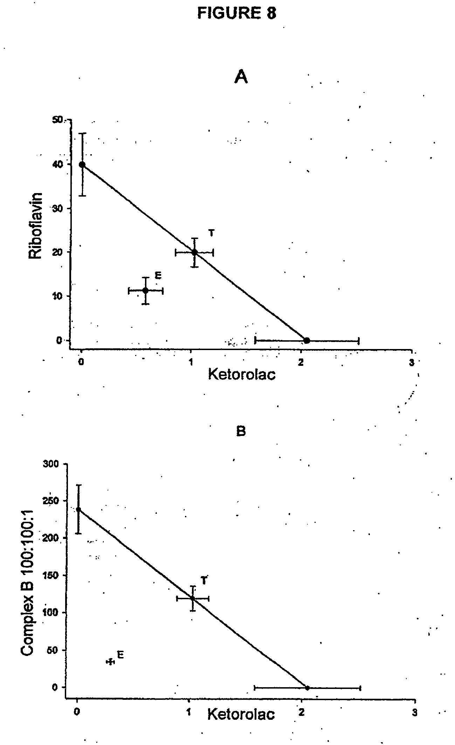

In Figure 8 the isobolograms show the additive effect of the ketorolac and riboflavin combination, as well as the synergic interaction of ketorolac and B-complex combination. Ketorolac and B-complex combination shows a surprisingly synergic interaction in the isobolograms, while ketorolac and riboflavin combination show an additive effect.

Horizontal and vertical bars indicate the SEM, the line connecting the X-axis with the Y-axis represents the line of the theoretical additive effect. The point in the middle of the line (T) represents the theoretical additive point estimated from the ED25 for both drugs. The (E) point under the line of the theoretical additive effect is the experimental point, which is of additive nature for ketorolac-riboflavin combination and of synergic nature for ketorolac-B-complex combination.

The estimated theoretical value for the ketorolac and B-complex combination was significantly higher than the obtained ED25 experimental value. In the combination of ketorolac and riboflavin there was no statistic difference related to the theoretical value.

### Conclusions:

Agents used show an anti-nociceptive effect.

It was demonstrated that ketorolac, riboflavin and B-complex are capable of reducing inflammatory pain.

We can surprisingly observe in the isobolograms that ketorolac and B-complex combination show a synergic interaction, the effect is greater than the sum of each agent's effects administered alone, while ketorolac-riboflavin combination shows an additive effect (the effect is equivalent to the sum of each agent's effect administered alone), being the most common observed effect.

### EXAMPLE 6: CLINICAL STUDY

In a study with ten post-cesarean patients under normal post-surgical evolution without base pathology and no allergy history to the formulation components.

A dose ketorolac (15 mg) and B-complex (Vitamin B1 100 mg, Vitamin B6 100 mg and Vitamin B12 5 mg) were intramuscularly administered to the patients.

The ten patients showed an improvement against pain after 30 minutes and could be moved from the operation room to a general room in the hospital, there were no local irritating reactions in the patient caused by the composition.

Results of the clinical studies reveal the existence of a synergic effect achieved with this composition.

Any person skilled in the technical field of this invention can be able to carry out modifications not described in this application. However, if any such change would need from the subject matter claimed in the following claims, then said composition will be included in the scope of the invention.

## Claims

1. A pharmaceutical composition for parenteral administration **characterized by** comprising the following two parenteral solution units:
Unit 1
| **Active ingredients and excipients.** | **Quantity in mg/ml** |
|---|---|
| Ketorolac tromethamine | 2.0 to 30.0 mg |
| Ethyl alcohol 96°GL | 85 to 115 mg |
| Anhydrous sodium dibasic phosphate | 28.5 to 38.5 mg |
| Citric acid | 1.1 to 1.5 mg |
| Water for injection | q.s. 1.0 ml |
| Total | 1.00 ml |
where the pH of the solution is 7.5 to 9.5; and
Unit 2
| **Active ingredients and excipients.** | **Quantity in mg/ml** |
|---|---|
| Thiamine chlorhydrate | 15 to 250 mg |
| Pyridoxine chlorhydrate | 15 to 250 mg |
| Cyanocobalmin | 0.1 to 10 mg |
| Disodium edentate | 0.08 to 0.115 mg |
| Propyl paraben | 0.068 to 0.092 mg |
| Methyl paraben | 0.52 mg to 1.08 mg |
| Sodium Hydroxide solution | 0.06 ml to 0.08 ml |
| Propylene glycol | 0.085 ml to 0.15 ml |
| Water for injection | q.s. 1.0 ml |
| Total | 1.0 ml |
where the pH of the solution is 2.5 to 4.5;
wherein the first and second units are mixed to form a composition in solution, with a final pH of 3.5 to 5.5.

## Patentansprüche

1. Pharmazeutische Zusammensetzung zur nicht oralen Anwendung, charakterisiert durch die folgenden zwei Lösungseinheiten zur nicht oralen Anwendung:
Einheit 1
| **Aktive Bestandteile und Exzipienten** | **Menge in mg/ml** |
|---|---|
| Ketorolactromethamin | 2,0 bis 30,0 mg |
| Ethylalkohol 96° GL | 85 bis 115 mg |
| Nicht wässriges dibasisches Natriumphosphat | 28,5 bis 38,5 mg |
| Zitronensäure | 1,1 bis 1,5 mg |
| Wasser zur Injektion | q.s. 1,0 ml |
| Gesamt | 1,00 ml |
wobei der pH der Lösung 7,5 bis 9,5 ist und
Einheit 2
| **Aktive Bestandteile und Exzipienten** | **Menge in mg/ml** |
|---|---|
| Thiaminchlorhydrat | 15 bis 250 mg |
| Pyridoxinchlorhydrat | 15 bis 250 mg |
| Cyanocobalmin | 0,1 bis 10 mg |
| Dinatriumedentat | 0,08 bis 0,115 mg |
| Propylparaben | 0,068 bis 0,092 mg |
| Methylparaben | 0,52 mg bis 1,08 mg |
| Natriumhydroxid-Lösung | 0,06 ml bis 0,08 ml |
| Propylenglycol | 0,085 ml bis 0,15 ml |
| Wasser zur Injektion | q.s. 1,0 ml |
| Gesamt | 1,0 ml |
wobei der pH der Lösung 2,5 bis 4,5 ist,
und wobei die erste und zweite Einheit gemischt werden, um eine lösliche Zusammensetzung zu bilden, mit einem endgültigen pH von 3,5 bis 5,5.

## Revendications

1. Composition pharmaceutique pour administration parentérale **caractérisée en ce qu'**elle comprend les deux unités de solutions parentérales suivantes :
Unité 1 :
| **Principes actifs et excipients.** | **Quantité en mg/ml** |
|---|---|
| kétorolac trométhamine | 2,0 à 30,0 mg |
| Alcool éthylique 96°GL | 85 à 115 mg |
| Phosphate dibasique de sodium anhydre | 28,5 à 38,5 mg |
| Acide citrique | 1,1 à 1,5 mg |
| Eau pour injection | q.s. 1,0 ml |
| Total | 1,00 ml |
où le PH de la solution est de 7,5 à 9,5 ; et
Unité 2 :
| **Principes actifs et excipients.** | **Quantité en mg/ml** |
|---|---|
| Chlorhydrate de thiamine | 15 à 250 mg |
| Chlorhydrate de pyridoxine | 15 à 250 mg |
| cyanocobalamine | 0,1 à 10 mg |
| Edétate disodique | 0,08 à 0,115 mg |
| Propyl parabène | 0,068 à 0,092 mg |
| Méthyl parabène | 0,52 mg à 1,08 mg |
| Solution d'Hydroxyde de Sodium | 0,06 ml à 0,08 ml |
| Propylène glycol | 0,085 ml à 0,15 ml |
| Eau pour injection | q.s. 1,0 ml |
| Total | 1,0 ml |
où le PH de la solution est de 2,5 à 4,5 ;
dans laquelle la première et la seconde unités sont mélangées pour former une composition en solution, avec un PH final de 3,5 à 5,5.
